(19) **Europäisches Patentamt / European Patent Office / Office européen des brevets**

(11) **EP 3 536 239 A1**

(12) **EUROPEAN PATENT APPLICATION**
published in accordance with Art. 153(4) EPC

(43) Date of publication:
**11.09.2019 Bulletin 2019/37**

(21) Application number: **17867941.1**

(22) Date of filing: **02.11.2017**

(51) Int Cl.:
**A61B 5/1455** (2006.01)  **G01N 33/49** (2006.01)

(86) International application number:
**PCT/JP2017/039754**

(87) International publication number:
**WO 2018/084249 (11.05.2018 Gazette 2018/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **04.11.2016 JP 2016216493**

(71) Applicant: **Kowa Company, Ltd.**
**Nagoya-shi, Aichi 460-8625 (JP)**

(72) Inventors:
• **ASANO, Takaharu**
 **Higashimurayama-shi**
 **Tokyo 189-0022 (JP)**
• **YABUSAKI, Katsumi**
 **Higashimurayama-shi**
 **Tokyo 189-0022 (JP)**

(74) Representative: **Ribeiro, James Michael**
 **Withers & Rogers LLP**
 **4 More London Riverside**
 **London SE1 2AU (GB)**

(54) **IN-BLOOD COMPONENT MEASUREMENT METHOD, IN-BLOOD COMPONENT MEASUREMENT DEVICE, AND IN-BLOOD COMPONENT MEASUREMENT PROGRAM**

(57)  Provided are an in-blood component measurement method, an in-blood component measurement device, and an in-blood component measurement program, enabling more accurate measurement of a proportion of glycohemoglobin in the total hemoglobin concentration in blood. The in-blood component measurement method is designed to have a constitution including the steps of irradiating, with near-infrared light, blood that is a target to be measured for a proportion of glycohemoglobin in the total hemoglobin concentration in blood, receiving transmitted light of the near-infrared light passing through the blood, measuring an absorption spectrum of the blood on the basis of the transmitted light received, and calculating the proportion for the blood that is the target of measurement by comparing absorbance at a first wavelength range including 1450 nm in the measured absorption spectrum to absorbance at the first wavelength range in the absorption spectrum of blood in which the proportion is already known.

FIG. 2

**Description**

[Field]

The present invention relates to an in-blood component measurement method, an in-blood component measurement device, and an in-blood component measurement program for measuring a proportion of glycohemoglobin in the total hemoglobin concentration in blood.

[Background]

Glycohemoglobin, also called HbAlc, occurs when an aldehyde group of glucose which has assumed a chain structure in aqueous solution bonds to a β-chain N-terminal amino group of hemoglobin. The HbAlc value represents a proportion of glycohemoglobin concentration in the total hemoglobin concentration in blood, and is an important indicator in diabetes diagnosis because it correlates with average blood glucose value when the blood glucose values of a test subject are observed over a period of months. Consequently, assessing the HbAlc value is important for diabetes prevention and early detection, and for follow-up after diabetes treatment, and various measurement methods have been proposed including high performance liquid chromatography (HPLC) methods and enzymatic methods or immune methods, but these methods are not easy because they require detection reagents and equipment such as columns and feed pumps. Moreover, the HbAlc value cannot be measured non-invasively because collected blood is required for measurement.

One example of a method for measuring the HbAlc value is a method of irradiating a living body with a plurality of monochromatic lights with different wavelengths, calculating the absorbance of the blood at each wavelength from the change width of the transmitted light quantity due to pulsation, and computing these to calculate the HbAlc value (see PTL 1). Another method is to irradiate a living body with two monochromatic lights in different wavelength ranges, calculate the absorbance of the blood at each wavelength from the change width of the transmitted light quantity due to pulsation, and compute these to calculate the HbAlc value (see PTL 2). Yet another method is to measure glucose using light absorbance at 1110 to 1560 nm and glycohemoglobin using light absorbance at 520 to 620 nm (see PTL 3). However, with these measurement methods it is difficult to measure the HbAlc value accurately because the methods use a wavelength range in which the change in absorbance caused by HbAlc is small.

Although NPL 1 describes that absorbance near 1450 nm changes when hemoglobin is glycosylated, moreover, it does not describe a method for measuring a proportion (HbAlc value) of glycohemoglobin in the total hemoglobin concentration in blood even if the total hemoglobin concentration changes.

Non-invasive methods of measuring blood components include a method of irradiating a living body with light at wavelengths of 600 to 1100 nm, and measuring the blood absorption spectrum non-invasively from the change width of the transmitted light quantity due to pulsation (PTL 4). However, this method cannot measure the HbAlc value since it cannot detect the light absorbance specific to HbAlc because the measured wavelength range is 1100 nm and lower. A similar method is a method of irradiating a living body with near-infrared light at wavelengths of 950 nm and above, and measuring the blood absorption spectrum non-invasively from the change width of the transmitted light quantity due to pulsation (PTL 5) . However, this is a method for measuring glucose and hemoglobin concentrations in blood using absorbance at 1000 to 1380 nm and 1620 to 1730 nm, and cannot measure the HbAlc value representing glucose bound to hemoglobin. Another non-invasive blood measurement uses the optical rotation and absorbance of near-infrared light at 1400 to 1550 nm (PTL 6), but this method is used to measure glucose, cholesterol, albumin or hemoglobin in blood, and cannot measure the HbAlc value.

Another similar method measures blood components non-invasively using the absorbance spectrum at 800 to 1850 nm (PTL 7), but this method measures hemoglobin, cholesterol, albumin and glucose concentrations in blood, and cannot measure the HbAlc value. Another method measures blood components non-invasively from absorbance of monochromatic light at the four wavelengths of 1525 nm, 1690 nm, 1575 nm and 1442 nm (PTL 8), but this method is used to measure blood glucose, and cannot measure the HbAlc value. A measurement method using optical coherence tomography (OCT) has been disclosed as a non-invasive method for measuring blood components (PTL 9), but this method uses light at 1300 to 1440 nm to measure blood glucose, and cannot measure the HbAlc value.

[Citation List]

[Patent Literature]

[PTL 1] JP-A-2005-253478
[PTL 2] JP-A-2004-248716
[PTL 3] European Patent No. 0631137
[PTL 4] JP-A-H07-088105
[PTL 5] WO 03/071939
[PTL 6] JP-A-2004-081284
[PTL 7] JP-B-3875798
[PTL 8] JP-A-H11-155840
[PTL 9] Japanese National Publication of International Patent Application No. 2008-546430

[Non Patent Literature]

[NPL 1] Laser Physics Letters, Vol. 5, 460-464 (2008), Monitoring of blood proteins glycation by refractive index and spectral measurements

[Summary]

[Technical Problem]

In light of these circumstances, the technology disclosed here aims to provide an in-blood component measurement device, an in-blood component measurement method, and an in-blood component measurement program capable of increasing the accuracy of measurement of a proportion of glycohemoglobin in the total hemoglobin concentration in blood.

[Solution to Problem]

The inventors of this application found as a result of exhaustive research that, when measuring the near-infrared absorption spectrum of blood, absorbance near 1450 nm varies to an unusual degree depending on the HbAlc value in comparison with other wavelengths. On the basis of the fact that absorbance near 900 to 1300 nm varies according to the total hemoglobin concentration, we also found that an absorbance measurement correlating with the HbAlc value could be obtained independently of the effect of total hemoglobin concentration by standardizing the absorption spectra or the ratio of the absorbance at both wavelengths, thus completing the in-blood component measurement device and the in-blood component measurement method of the technology disclosed here.

In the in-blood component measurement method disclosed here, blood that is a target to be measured, in which the proportion (HbAlc value) of glycohemoglobin in the total hemoglobin concentration in blood is unknown, is irradiated with near-infrared light; transmitted light of the near-infrared light passing through the blood is received; an absorption spectrum of the blood is measured on the basis of the transmitted light received; and the proportion for the blood that is the target of measurement is calculated by comparing absorbance at a wavelength range including 1450 nm in the measured absorption spectrum to absorbance at the wavelength range in the absorption spectrum of blood in which the proportion is already known. In this method, the accuracy of measurement of the proportion can be increased because the method focuses on a wavelength range that fluctuates more depending on the proportion (HbAlc value) of glycohemoglobin in the total hemoglobin concentration in blood out of the wavelength ranges of the absorption spectrum.

The absorbance of the measured absorption spectrum may also be standardized by absorbance at another wavelength range prior to the comparing. This causes the variation in the proportion at the wavelength range including 1450 nm to appear more accurately independently of the total hemoglobin concentration. Absorbance at the other wavelength range may be absorbance contained in the wavelength range of 900 nm to 1300 nm. Moreover, the wavelength range for measurement is 1300 nm to 1500 nm, or preferably 1400 nm to 1500 nm, or more preferably 1430 nm to 1470 nm.

The in-blood component measurement device disclosed here includes an irradiator that irradiates, with near-infrared light, blood that is a target to be measured for a proportion of glycohemoglobin in the total hemoglobin concentration in blood; a light receiver that receives transmitted light of the near-infrared light passing through the blood; a measuring unit that measures an absorption spectrum of the blood on the basis of the transmitted light received; and a calculating unit that calculates the proportion for the blood that is the target of measurement by comparing absorbance at a wavelength range including 1450 nm to absorbance at that the wavelength range in the absorption spectrum of blood in which the proportion is already known. Prior to the comparing, the calculating unit may also standardize the absorbance of the measured absorption spectrum by absorbance at another wavelength range. The absorbance at the other wavelength may be absorbance contained in a wavelength range of 900 nm to 1300 nm. The wavelength range for measurement is 1300 nm to 1500 nm, or preferably 1400 nm to 1500 nm, or more preferably 1430 nm to 1470 nm.

The in-blood component measurement program disclosed here causes a computer to perform: measuring an absorption spectrum of blood on the basis of near-infrared light passing through the blood that is a target to be measured for a proportion of glycohemoglobin in the total hemoglobin concentration in blood; and calculating the proportion in the blood that is the target of measurement by comparing absorbance at a wavelength range including 1450 nm in the measured absorption spectrum to absorbance at the wavelength range in the absorption spectrum of blood in which the proportion is already known. Prior to the comparing, the absorbance of the measured absorption spectrum may also be standardized by absorbance at another wavelength range on the computer. The absorbance at the other wavelength range may be absorbance contained in a wavelength range of 900 nm to 1300 nm. The wavelength range for measurement is 1300 nm to 1500 nm, or preferably 1400 nm to 1500 nm, or more preferably 1430 nm to 1470 nm.

[Advantageous Effects of Invention]

According to the technology disclosed here, it is possible to provide an in-blood component measurement device, an in-blood component measurement method, and an in-blood component measurement program whereby the accuracy of measurement of the proportion of glycohemoglobin in the total hemoglobin concentration in blood can be increased.

[Brief Description of Drawings]

Fig. 1 illustrates an outline of an in-blood component measurement device of one embodiment.
Fig. 2 illustrates a schematic view of an in-blood component measurement device of one embodiment.
Fig. 3 illustrates a schematic view of one portion of an in-blood component measurement device of one embodiment.
Fig. 4 illustrates one example of a flowchart of processing performed by an in-blood component measurement device of one embodiment.
Fig. 5 illustrates one example of absorption spectra of hemoglobin solutions measured in one embodiment.
Fig. 6 illustrates one example of the difference between the absorption spectra of hemoglobin solutions measured in one embodiment and an absorption spectrum obtained from the solvent of the hemoglobin solutions.
Fig. 7 illustrates one example of absorbance at 948 nm in a difference spectrum of one embodiment plotted against total hemoglobin concentration.
Fig. 8 illustrates one example of HbAlc values obtained with a measurement kit of one embodiment from erythrocyte suspensions.
Fig. 9 illustrates one example of the absorption spectra of erythrocytes with different HbAlc values in one embodiment.
Fig. 10 illustrates one example of a graph obtained by standardizing the graph of HbAlc values illustrated in Fig. 9 by a wavelength range of 900 to 1300 nm.
Fig. 11 illustrates one example of absorbance at 1439 nm plotted against HbAlc values for standardized spectra of one embodiment.
Fig. 12 illustrates one example of absorption spectra of hemoglobin solutions with different HbAlc values in one embodiment.
Fig. 13 illustrates one example of a graph obtained by standardizing each of the HbAlc values in the graph of Fig. 12 by a wavelength range of 900 to 1300 nm.
Fig. 14 illustrates one example of absorbance at 1439 nm plotted against HbAlc values using standardized HbAlc values of one embodiment.
Fig. 15 illustrates one example of absorption spectra of hemoglobin solutions with different total hemoglobin concentrations in one embodiment.
Fig. 16 illustrates one example of absorbance at 1021 nm and 1439 nm plotted against total relative hemoglobin concentration in one embodiment.
Fig. 17 illustrates one example of a graph obtained by standardizing each HbAlc value in the graph of Fig. 15 by the wavelength range of 900 to 1300 nm.

Fig. 18 illustrates one example of standardized absorbance at 1021 nm and 1439 nm plotted against total relative hemoglobin concentration in one embodiment.

[Description of Embodiments]

An embodiment of the invention is explained below with reference to the drawings. In the explanations below, an HbAlc value means a value representing the proportion of glycohemoglobin in the total hemoglobin concentration in blood. One example of glycohemoglobin is hemoglobin produced when the aldehyde groups of glucose which has assumed a chain structure in aqueous solution bond to the β-chain N-terminal amino groups of hemoglobin.

The organism that is the object of HbAlc value measurement in the present embodiment need not be a human since hemoglobin and glycohemoglobin are contained in the blood of a wide range of animals including humans. The hemoglobin and glycohemoglobin used in preparing the calibration curves are preferably derived from the same organism as the object of measurement. Specific examples of organisms to be measured include fish and shellfish such as eels, flounder, koi, sea bream, schrenck's limpets, shrimp and clams, birds such as chickens, quail, ostrich and pigeons, and mammals such as humans, monkeys, sheep, cows, pigs, goats, horses, mice, dogs, cats, deer and seals. Of these, birds and mammals are preferred as objects of measurement, and humans are more desirable from the perspective of preventing and treating diabetes.

Measurement of the near-infrared absorption spectrum of a hemoglobin solution in the present embodiment is explained. Freeze-dried hemoglobin powder (Wako Pure Chemical Industries, 151234) is used as the hemoglobin. Saline (Otsuka Saline, 035-08151-7) is used as the solvent for dissolving the hemoglobin.

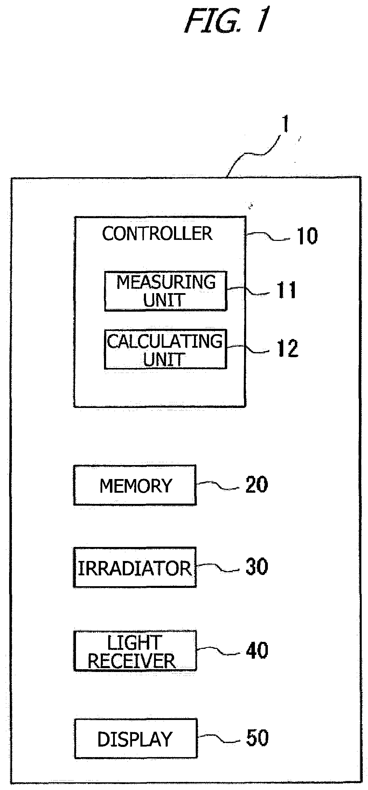

Fig. 1 illustrates one example of an outline of an in-blood component measurement device 1 in the present embodiment. In in-blood component measurement device 1, for example a part of the body (finger, etc.) of a test subject who is the object of HbAlc value measurement is irradiated with near-infrared light, and the HbAc1 value is calculated using a blood absorption spectrum obtained from the transmitted light. As illustrated in Fig. 1, in-blood component measurement device 1 has controller 10, memory 20, irradiator 30, light receiver 40 and display 50.

The controller 10 controls the operation of each part within the in-blood component measurement device 1. The memory 20 stores the programs for executing the various processes in the in-blood component measurement device 1 as explained below. Data about previously-prepared calibration curves representing the relationship between absorbance and HbAlc value are also stored in the memory 20. The memory 20 also stores data obtained when executing the various processes in the in-blood component measurement device 1. The controller 10 executes the various processes in the in-blood component measurement device 1 by developing the programs stored in the memory 20 into the Random Access Memory (RAM; not illustrated) of the device and running the developed programs. The irradiator 30 irradiates near-infrared light on a part (finger, etc.) of the body of a test subject who is the object of in-blood component measurement. The transmitted light passing through the body of the test subject is received by the light receiver 40.

The controller 10 also has a measuring unit 11 and calculating unit 12 as part of the function of the controller 10. The measuring unit measures the absorption spectrum of the blood of a test subject on the basis of the transmitted light received by the light receiver 40. The calculating unit 12 calculates the HbAc1 value of the measured blood by comparing absorbance at a wavelength range including 1450 nm in the measured absorption spectrum with absorbance at the same wavelength range in the absorption spectrum of blood with a known HbAlc value. The HbAlc value calculation results obtained by the calculating unit 12 are displayed on the display 50.

In the present embodiment, a multichannel Fourier transform spectrometer for example is used as the light receiver 40 for measuring the near-infrared absorption spectrum. In this device, incoming light is separated by a Savart plate, an interference pattern (interferogram) resulting from interference generated between the separated lights with a Fourier lens is obtained by a line sensor, and the resulting interferogram is Fourier transformed to obtain an optical spectrum. The measurable wavelengths with a multichannel Fourier transform spectrometer include the entire near-infrared range (900 to 2500 nm). By using a multichannel Fourier transform spectrometer to measure the transmitted light spectrum of a specimen, and then comparing it to a blank transmitted light spectrum obtained in advance, it is possible to obtain an absorption spectrum of the transmitted light spectrum of the specimen relative to a blank.

In the present embodiment, hemoglobin powder is dissolved in saline to prepare multiple hemoglobin solutions with different total hemoglobin concentrations (0, 10, 15, 20 and 25 g/dL). The prepared hemoglobin solutions are sealed in quartz cells with optical path lengths of 1 mm, and the near-infrared absorption spectra of the hemoglobin solutions are measured with a multichannel Fourier transform spectrometer (blank: empty cell).

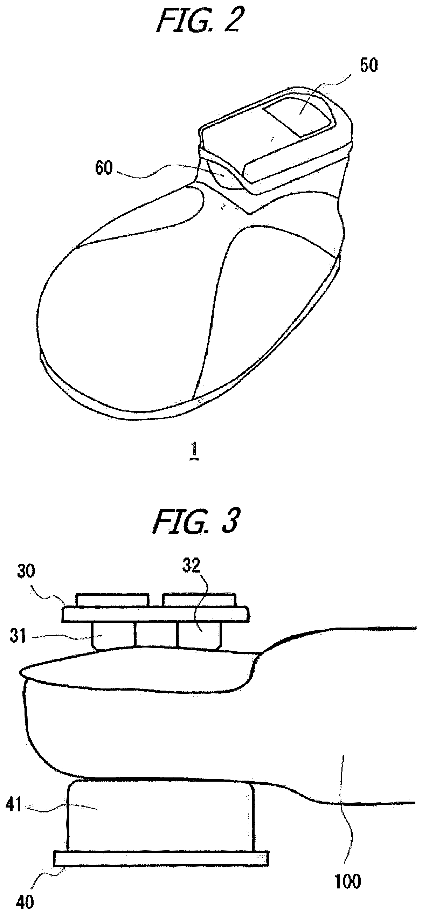

Fig. 2 illustrates a schematic view of one example of an in-blood component measurement device 1 of the present embodiment. In-blood component measurement device 1 is provided with an opening 60 for inserting a finger 100 of a test subject. An irradiator 30 and light receiver 40 are provided inside the opening 60. Fig. 3 is a schematic view illustrating the finger 100 of the test subject inserted into the opening 60 in the in-blood component measurement device 1 of the Fig. 2. The irradiator 30 and light receiver 40 are disposed on either side of the test subject's finger 100 inserted into the opening 60.

The irradiator 30 has light-emitting diodes (LEDs) 31 and 32. As one example, the wavelengths of the light emitted by the LEDs 31 and 32 are 940 nm that is a wavelength for normalization and 1450 nm that is a wavelength for measurement, respectively. However, the number and irradiation wavelengths of the LEDs provided in the irradiator 30 are not limited to these. Moreover, light receiver 40 has light detector 41. Thus, near-infrared light from the irradiator 30 irradiating the finger 100 is received by the light receiver 40 after passing through the finger 100. In the in-blood component measurement device 1, the HbAlc value is calculated by the process explained below on the basis of the near-infrared light received by the light receiver 40. The calculated HbAlc value is then displayed on the display 50.

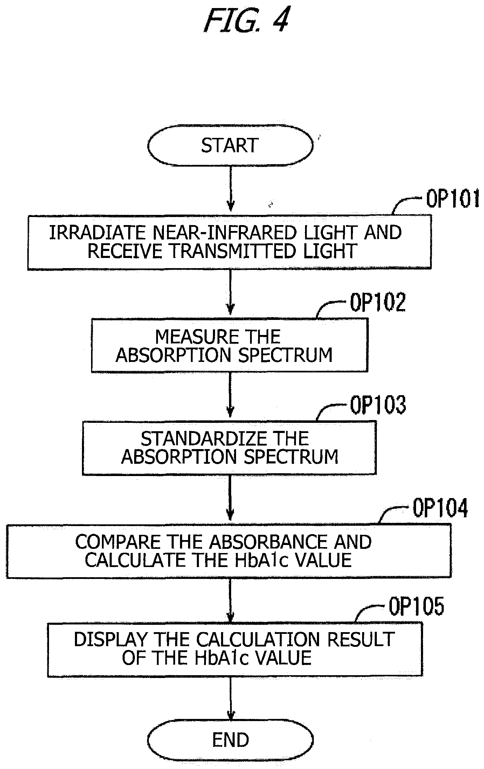

Fig. 4 illustrates one example of a flow chart of the operations executed by the controller 10. The controller 10 initiates the operations illustrated on the flow chart of Fig. 4 according to the operations of the user of the in-blood component measurement device 1 for example.

In OP101, the controller 10 controls the irradiator 30 to irradiate a part of the body of a test subject with near-infrared light. The irradiating near-infrared light passes through the body of the test subject, and enters light receiver 40 as transmitted light. The operation next progresses to OP102. In OP102, the controller 10 performs the absorption spectrum measurement described above using the transmitted light received by the light receiver 40.

Next, in OP103 controller 10 uses the measured absorption spectrum of blood with a known HbAlc value stored in the memory 20 to standardize the absorption spectrum measured in OP102. In OP104, the controller 10 further compares the absorbance of the absorption spectrum standardized in OP103 with the absorbance of an absorption spectrum measured in blood with a known HbAlc value, and calculates the HbAlc value of the test subject's blood. In OP105, the controller 10 displays the HbAlc value calculated in OP104 on the display 50 to thereby complete the operations of the flow chart.

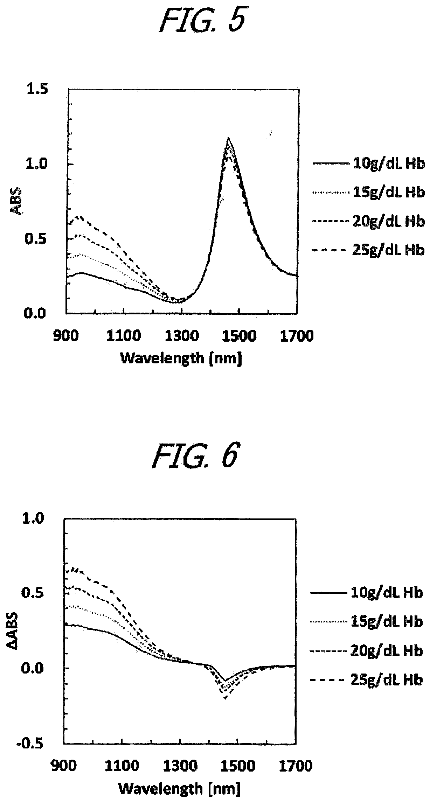

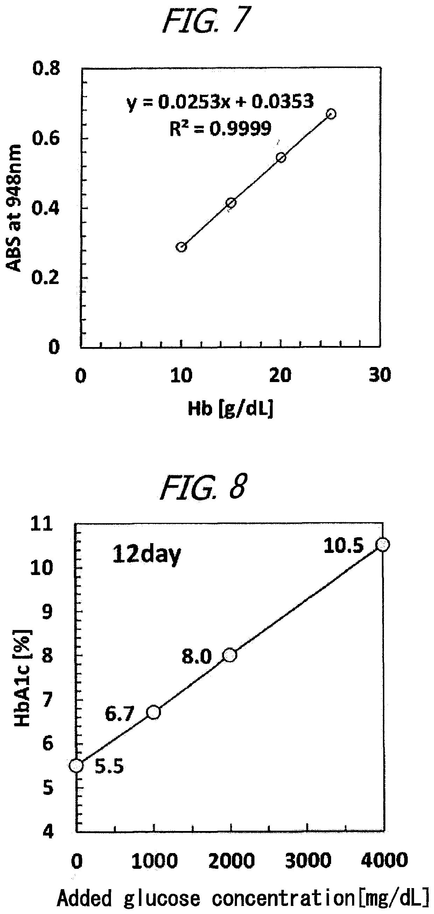

Next, HbAlc value measurement in the present embodiment is explained in detail. Fig. 5 illustrates one example of the absorption spectra of hemoglobin solutions measured in the present embodiment. The wavelength is depicted along the horizontal axis in the graph of Fig. 5, and the absorbance on the vertical axis. As illustrated in Fig. 5, the absorption spectra of the hemoglobin solutions have absorbance peaks near 1450 nm attributable to water ($H_2O$), and the absorbance near 900 to 1300 nm increases depending on the total hemoglobin concentration. Fig. 6 illustrates one example of difference spectra illustrating the difference between the absorption spectra of the measured hemoglobin solutions and the absorption spectrum of saline obtained in advance as the solvent of a hemoglobin solution. In the graph of Fig. 6, the wavelength is depicted along the horizontal axis, and the difference in absorbance on the vertical axis. Fig. 7 illustrates one example of the results of a plot of the absorbance at 948 nm in the difference spectrum plotted against total hemoglobin concentration. In the graph of Fig. 7, total hemoglobin concentration is depicted along the horizontal axis and absorbance on the vertical axis. As illustrated in Fig. 7, the total hemoglobin concentration tends to be greater in proportion to the absorbance at 900 to 1300 nm.

Next, near-infrared absorption spectrum spectrum measurement of a suspension of erythrocytes containing glycohemoglobin in the present embodiment is explained. Blood (about 50 mL) collected from a healthy subject and including about 200 μL (about 0.4% of blood volume) of heparin (AY Pharma, heparin sodium injection N 10,000 units/10 mL "AY") added thereto as anticoagulant is used as the blood. D(+)-glucose (Wako Pure Chemical Industries, 045-31162) is used as the glucose added to glycosylate the hemoglobin. Saline (Otsuka Saline, 035-08151-7) is used as the washing solution for the erythrocytes and the solvent for the glucose. A DCA2000 system with a DCA2000 HbAlc cartridge (Made by Siemens) is used as a measurement kit for obtaining HbAlc value reference values. When using this kit, about 1 μL of a hemoglobin-containing (7 to 24 g/dL) specimen solution corresponding to total blood is collected by the specialized cartridge, and the HbAlc value is calculated on the basis of the latex immunoagglutination inhibition method.

The centrifugation conditions for precipitating the erythrocytes are 10 minutes at about 4°C at a rotation of 3000 rpm. After the blood is centrifuged, the precipitated erythrocytes are collected and saline is added to obtain the original volume. This process is repeated twice to eliminate components other than erythrocytes and prepare an erythrocyte suspension.

For the glucose stock solution that is added to glycosylate the hemoglobin, glucose is dissolved in saline to prepare aqueous glucose solutions with glucose concentrations of 0, 5, 10 and 20 g/dL. The erythrocyte suspension is divided into four (4 mL), and 1 mL of each of the aqueous glucose solutions prepared with different concentrations is added to a different erythrocyte suspension. That is, the final concentrations of glucose in the erythrocyte suspensions are 0, 1000, 2000 and 4000 mg/dL. Each erythrocyte suspension with the added aqueous glucose solution is left for 12 days at room temperature and then centrifuged, after which the precipitated erythrocytes are collected and saline is added to obtain the original volume. This process is repeated twice to eliminate the added glucose from the erythrocyte suspensions. Part of each of the erythrocyte suspensions is then centrifuged to precipitate the erythrocytes, which are collected and enclosed in a quartz cell with an optical path length of 0.1 mm, and the near-infrared absorption spectra are measured with a multichannel Fourier transform spectrometer (blank: empty cell). Normalization of the spectra is performed using the Standard Normal Variate (SNV) conversion represented by Formula (1) below.
[Math. 1]

$$Z_i = \frac{x_i - ave}{sd} \cdot \cdot \cdot (1)$$

In the formula, $x_i$ is the spectrum value before normalization, $Z_i$ is the spectrum value after normalization, ave is the average of the $x_i$ values at 900 to 1300 nm, and sd is the standard deviation of $x_i$ at 900 to 1300 nm. In Formula (1), i is a natural number indicating the elements of each near-infrared absorption spectrum to be standardized. That is, because in the example above the absorption spectrum at 900 to 1300 nm is composed of 41 wavelengths, i = 1 to 41. The range of i can be determined appropriately according to the wavelength resolution of the spectrometer used to measure the near-infrared absorption spectra.

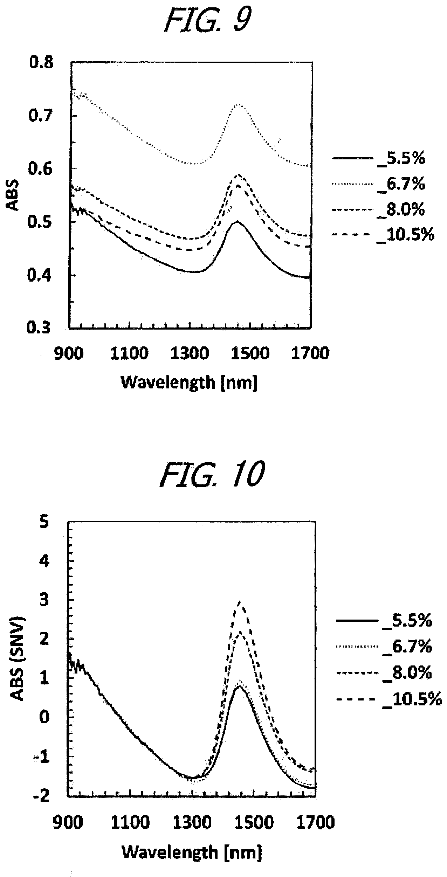

The remainder of each erythrocyte suspension is supplied to the measurement kit described above to obtain an HbAlc value. Fig. 8 illustrates one example of the HbAlc values obtained with this measurement kit from each erythrocyte suspension. In Fig. 8, the added glucose concentration is depicted along the horizontal axis, and the HbAlc value on the vertical axis. From the graph of Fig. 8, it can be seen that in the present embodiment it is possible to prepare erythrocyte suspensions with HbAlc values that differ according to the added glucose concentration. Moreover, Fig. 9 illustrates one example of the absorption spectra of erythrocytes with different HbAlc values. In Fig. 9, the wavelength is depicted along the horizontal axis, and the absorbance on the vertical axis. The reason why the offsets of the spectral shapes differ at each HbAlc value as illustrated in Fig. 9 is that the total hemoglobin concentrations vary in the process of collecting the erythrocytes by centrifugation and washing the collected erythrocytes.

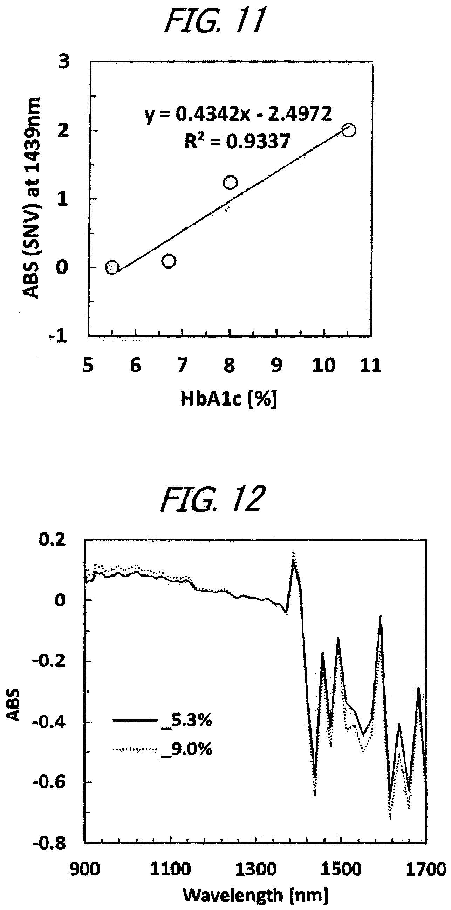

In the present embodiment, absorbance near 1450 nm is standardized on the basis of absorbance at a wavelength range of 900 to 1300 nm in order to obtain spectra with a uniform total hemoglobin concentration with respect to the spectral shape of each HbAlc value. Fig. 10 illustrates one example of a graph obtained by standardizing the graph of HbAlc values illustrated in Fig. 9 on the basis of the wavelength range of 900 to 1300 nm. In Fig. 10, the wavelength is depicted along the horizontal axis and standardized absorbance on the vertical axis. It can be seen from the graph of Fig. 10 that in the standardized spectra, absorbance near 1450 nm in particular is greater depending on the HbAlc value. Fig. 11 illustrates one example of results obtained by plotting absorbance at 1439 nm against the HbAlc values for the standardized spectra. In the graph of Fig. 11, the HbAlc value is depicted along the horizontal axis and standardized absorbance on the vertical axis. It can be seen from the graph of Fig. 11 that absorbance at 1439 nm correlates well with the HbAlc value.

That is, in the present embodiment the absorption spectrum is measured using blood samples with known HbAlc values. Absorbance in the range of 900 to 1300 nm is then used to standardize absorbance near 1450 nm, a calibration curve representing the relationship between the standardized absorbance near 1450 nm and the HbAlc value is prepared in advance, and the absorption spectrum of a blood sample with an unknown HbAlc value is then measured in the same way. The measured absorption spectrum is also standardized in the same way as the blood sample with the known HbAlc value to obtain absorbance near 1450 nm. The absorbance value thus obtained can then be compared to the prepared calibration curve to calculate the HbAlc value.

The absorbance changes of the glycohemoglobin in the present embodiment above are explained next. In one example, two erythrocyte suspensions with different HbAlc values (HbAlc = 5.3% and 9.0%) are centrifuged, and a supernatant containing hemoglobin dissolved by hemolysis is collected. This supernatant that is hemoglobin solution is passed through a filter with a pore diameter of 0.2 $\mu$m to remove insoluble matter, and enclosed in a polystyrene cell with an optical path length of 10 mm. The near-infrared absorption spectrum is then measured (blank: saline) with a multichannel Fourier transform spectrometer. The spectrum is standardized using the SNV conversion represented by Formula (1).

Fig. 12 illustrates one example of the absorption spectra of hemoglobin solutions with different HbAlc values. In the graph of Fig. 12, wavelength is depicted along the horizontal axis and absorbance on the vertical axis. In each case, as illustrated in Fig. 12, the solution is shown to contain hemoglobin because absorbance near 900 to 1300 nm is high. The total hemoglobin concentration of the hemoglobin solution with an HbAlc value of 9.0% is shown to be higher than that of the hemoglobin solution with an HbAlc value of 5.3%.

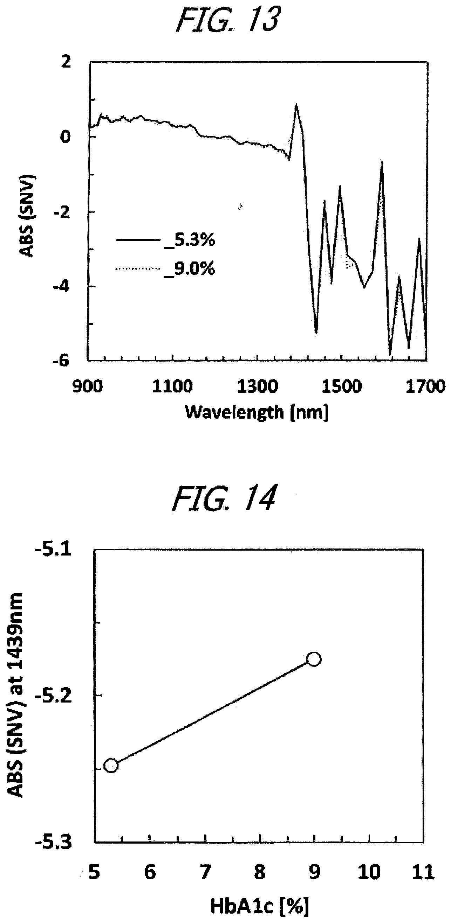

Fig. 13 illustrates one example of a graph obtained by standardizing the graph of HbAlc values illustrated in Fig. 12 on the basis of a wavelength range of 900 to 1300 nm as in Fig. 10. In Fig. 13, the wavelength is depicted along the horizontal axis, and the standardized absorbance on the vertical axis. Fig. 14 illustrates one example of the results of a plot of standardized absorbance at 1439 nm against HbAlc value. In Fig. 14, the HbAlc value is depicted along the horizontal axis and standardized absorbance on the vertical axis. It can be seen from Figs. 13 and 14 that in a hemoglobin solution, absorbance near 1450 nm in the standardized spectrum increases as the HbAlc value increases. This means that the increase in absorbance depending on the HbAlc value in erythrocytes derives from glycosylation of hemoglobin.

That is, according to the in-blood component measurement device 1 of the present embodiment, the HbAlc value can be calculated accurately on the basis of absorbance at a wavelength (near 1450 nm) at which the change in absorbance attributable to HbAlc is relatively great as the HbAlc value fluctuates.

Next, the relationship between total hemoglobin concentration and HbAlc value in the HbAlc value calculation of the present embodiment is explained. In one example, a hemoglobin solution (HbAlc value = 5.3%) prepared from an erythrocyte suspension is diluted 1.5x and 2x with saline to obtain diluted hemoglobin solutions. Given 1 as the total hemoglobin concentration of the hemoglobin solution before dilution, the total relative hemoglobin concentrations of the 1.5x dilution and 2x dilution are 0.67 and 0.5. However, the HbAlc value of the hemoglobin solution is the same before and after dilution. In the present embodiment, each diluted hemoglobin solution is enclosed in a polystyrene cell with an optical path length of 10 mm, and the near-infrared absorption spectrum (blank: saline) is measured using a multichannel Fourier transform spectrometer. The spectrum is standardized using the SNV conversion represented by Formula (1).

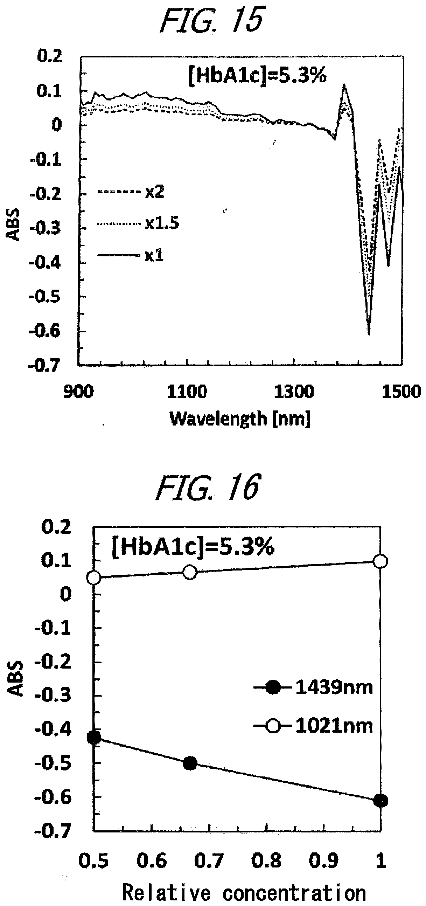

Fig. 15 illustrates one example of the absorption spectra of hemoglobin solutions with different total hemoglobin concentrations (HbAlc value 5.3% in each case). In Fig. 15, the wavelength is depicted along the horizontal axis, and absorbance on the vertical axis. Fig. 16 illustrates one example of the results of a plot of absorbance at 1021 nm and 1439 nm against total relative hemoglobin concentration. In the graph of Fig. 16, total relative hemoglobin concentration is depicted along the horizontal axis, and absorbance on the vertical axis. Absorbance at 1021 nm rises as the total relative hemoglobin concentration rises, while absorbance at 1439 nm declines. This shows that the relative concentration of water declines as the total hemoglobin concentration increases, and that absorbance near 1450 nm which includes absorption from water ($H_2O$) declines as a result.

Consequently, when calculating the HbAlc value using absorbance at 1439 nm in hemoglobin solutions with the same HbAlc value and different total relative hemoglobin concentrations, the HbAlc value calculated for each solution fluctuates depending on the total relative hemoglobin concentration. In the present embodiment, therefore, absorbance near 1450 nm is standardized on the basis of absorbance at a wavelength range of 900 to 1300 nm in order to obtain a spectrum with a uniform total hemoglobin concentration with respect to the spectral shape of each HbAlc value.

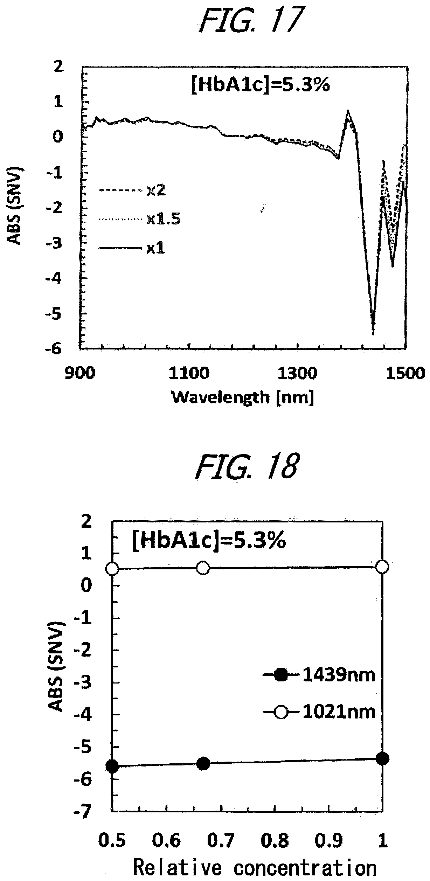

Fig. 17 illustrates one example of a graph obtained by standardizing absorbance near 1450 nm for each hemoglobin solution in the graph of Fig. 15 on the basis of absorbance at a wavelength range of 900 to 1300 nm. In Fig. 17, the wavelength is depicted along the horizontal axis and standardized absorbance on the vertical axis. Fig. 18 illustrates one example of the results of a plot of standardized absorbance at 1021 nm and 1439 nm against total relative hemoglobin concentration. In Fig. 18, the total relative hemoglobin concentration is depicted along the horizontal axis, and the standardized absorbance on the vertical axis. As illustrated in Fig. 18, absorbance at both 1021 nm and 1439 nm remains the same independent of total relative hemoglobin concentration.

That is, according to the in-blood component measurement device 1 of the present embodiment, the HbAlc value can be measured more accurately upon calculation of HbAlc values on a plurality of hemoglobin solution samples in comparison with conventional techniques even when the total relative hemoglobin concentration of the hemoglobin solutions are different by standardizing spectra to spectra with a uniform total hemoglobin concentration before calculating the HbAlc value using absorbance at 1439 nm.

The explanations above relate to the present embodiment, but the configuration of the in-blood component measurement device 1 above, the normalization methods and the method for measuring the HbAlc value are not limited to the embodiment described above, and various changes are possible to the extent that these conform to the technical idea of the invention.

For example, the in-blood component measurement device 1 calculates the HbAlc value non-invasively, but the HbAlc value can also be calculated accurately by the processes described above even if the device is configured to calculate the HbAlc value invasively. Also, for example the absorption spectrum measured in the embodiment described above may be a so-called whole-blood absorption spectrum obtained from collected blood, or an absorption spectrum of erythrocytes collected from whole blood, or a blood absorption spectrum extracted non-invasively using reflected light or transmitted light from a biological sample. Moreover, the measurement device used to measure the absorption spectrum may be equipped with a spectrometer that obtains absorbance at multiple wavelengths simultaneously, such as a multichannel Fourier transform spectrometer, or a single channel Fourier transform spectrometer, or a multichannel dispersive spectrometer, or a single channel dispersive spectrometer. Because the HbAlc value measurement method of the embodiment described above can be implemented without a detection reagent, the HbAlc value can be measured non-invasively using a blood absorption spectrum obtained non-invasively.

Moreover, in the absorption spectrum used in the measurement method of the embodiment described above the wavelength or wavenumber axis may be subjected to first-order or quadratic differentiation. The method of standardizing the spectrum may use a value obtained by dividing the absorbance near 1450 nm by absorbance at a wavelength selected from the wavelength range of 900 to 1300 nm, or a value obtained by applying normalization with a minimum value of 0 and a maximal value of 1 in any wavelength range between 900 and 1300 nm to absorbance near 1450 nm, or a value obtained by applying normalization involving SNV conversion at any wavelength range between 900 and 1300 nm to absorbance near 1450 nm.

The wavelength range of the absorbance used for normalization may be any in the range of 400 to 1300 nm at which hemoglobin absorbance is recognized, but is preferably 600 to 1300 nm, or more preferably 800 to 1300 nm, and 900 to 1300 nm is still more desirable from the standpoint of HbAlc value calculation accuracy. The wavelength range of the absorbance used in calculating the HbAlc value includes 1450 nm, and is preferably 1300 to 1500 nm, or more preferably 1400 to 1500 nm, or still more preferably 1430 to 1470 nm.

[Reference Signs List]

1     in-blood component measurement device
10    controller
11    measuring unit
12    calculating unit
20    memory
30    irradiator
40    light receiver

**Claims**

1. An in-blood component measurement method, the method comprising:

    irradiating, with near-infrared light, blood that is a target to be measured for a proportion of glycohemoglobin in the total hemoglobin concentration in blood;
    receiving transmitted light of the near-infrared light passing through the blood;
    measuring an absorption spectrum of the blood on the basis of the transmitted light received; and
    calculating the proportion for the blood that is the target of measurement by comparing absorbance at a first wavelength range including 1450 nm in the measured absorption spectrum to absorbance at the first wavelength range in the absorption spectrum of blood in which the proportion is already known.

2. The in-blood component measurement method according to Claim 1, wherein the absorbance at the first wavelength range is standardized on the basis of absorbance at a second wavelength range of the measured absorption spectrum prior to the comparing.

3. The in-blood component measurement method according to Claim 1 or 2, wherein the first wavelength range is 1300 to 1500 nm, or preferably 1400 to 1500 nm, or more preferably 1430 to 1470 nm.

4. The in-blood component measurement method according to Claim 2 or Claim 3 citing Claim 2, wherein the absorbance at the second wavelength range is absorbance included in the wavelength range of 900 nm to 1300 nm.

5. An in-blood component measurement device, comprising:

an irradiator that irradiates, with near-infrared light, blood that is a target to be measured for a proportion of glycohemoglobin in the total hemoglobin concentration in blood;
a light receiver that receives transmitted light of the near-infrared light passing through the blood;
a measuring unit that measures an absorption spectrum of the blood on the basis of the transmitted light received; and
a calculating unit that calculates the proportion for the blood that is the target of measurement by comparing absorbance at a first wavelength range including 1450 nm to absorbance at the first wavelength range in the absorption spectrum of blood in which the proportion is already known.

6. The in-blood component measurement device according to Claim 5, wherein the calculating unit standardizes the absorbance at the first wavelength range on the basis of absorbance at a second wavelength range of the measured absorption spectrum prior to the comparing.

7. The in-blood component measurement device according to Claim 5 or 6, wherein the first wavelength range is 1300 to 1500 nm, or preferably 1400 to 1500 nm, or more preferably 1430 to 1470 nm.

8. The in-blood component measurement device according to Claim 6 or Claim 7 citing Claim 6, wherein the absorbance at the second wavelength range is absorbance included in the wavelength range of 900 nm to 1300 nm.

9. An in-blood component measurement program, the program causing a computer to perform:

measuring an absorption spectrum of blood on the basis of near-infrared light passing through the blood that is a target to be measured for a proportion of glycohemoglobin in the total hemoglobin concentration in blood; and
calculating the proportion in the blood that is the target of measurement by comparing absorbance at a first wavelength range including 1450 nm in the measured absorption spectrum to absorbance at the first wavelength range in the absorption spectrum of blood in which the proportion is already known.

10. The in-blood component measurement program according to Claim 9, the program further causing the computer to perform standardizing the absorbance at the first wavelength range on the basis of absorbance at a second wavelength range in the measured absorption spectrum prior to the comparing.

11. The in-blood component measurement program according to Claim 9 or 10, wherein the first wavelength range is 1300 to 1500 nm, or preferably 1400 to 1500 nm, or more preferably 1430 to 1470 nm.

12. The in-blood component measurement program according to Claim 10 or Claim 11 citing Claim 10, wherein the absorbance at the second wavelength range is absorbance included in the wavelength range of 900 nm to 1300 nm.

## FIG. 1

1

CONTROLLER — 10

MEASURING UNIT — 11

CALCULATING UNIT — 12

MEMORY — 20

IRRADIATOR — 30

LIGHT RECEIVER — 40

DISPLAY — 50

## FIG. 2

## FIG. 3

# FIG. 4

```
           ┌─────────────┐
           │    START    │
           └──────┬──────┘
                  │
                  ▼                            ┌─OP101
    ┌─────────────────────────────────────┐
    │ IRRADIATE NEAR-INFRARED LIGHT AND    │
    │     RECEIVE TRANSMITTED LIGHT        │
    └─────────────────┬───────────────────┘
                      │
                      ▼                  ┌─OP102
           ┌──────────────────────┐
           │     MEASURE THE       │
           │ ABSORPTION SPECTRUM   │
           └──────────┬───────────┘
                      │
                      ▼                  ┌─OP103
           ┌──────────────────────┐
           │   STANDARDIZE THE     │
           │ ABSORPTION SPECTRUM   │
           └──────────┬───────────┘
                      │
                      ▼                        ┌─OP104
    ┌─────────────────────────────────────┐
    │  COMPARE THE ABSORBANCE AND          │
    │   CALCULATE THE HbA1c VALUE          │
    └─────────────────┬───────────────────┘
                      │
                      ▼                        ┌─OP105
    ┌─────────────────────────────────────┐
    │ DISPLAY THE CALCULATION RESULT       │
    │      OF THE HbA1c VALUE              │
    └─────────────────┬───────────────────┘
                      │
                      ▼
           ┌─────────────┐
           │     END     │
           └─────────────┘
```

## FIG. 5

## FIG. 6

## FIG. 7

$y = 0.0253x + 0.0353$
$R^2 = 0.9999$

ABS at 948nm vs Hb [g/dL]

## FIG. 8

12day

10.5

8.0

6.7

5.5

HbA1c [%] vs Added glucose concentration[mg/dL]

## FIG. 9

## FIG. 10

## FIG. 11

$y = 0.4342x - 2.4972$
$R^2 = 0.9337$

ABS (SNV) at 1439nm

HbA1c [%]

## FIG. 12

ABS

_____5.3%

············_9.0%

Wavelength [nm]

## FIG. 13

## FIG. 14

## FIG. 15

## FIG. 16

## FIG. 17

## FIG. 18

<div style="text-align:center">**INTERNATIONAL SEARCH REPORT**</div>

| International application No. |
| --- |
| PCT/JP2017/039754 |

| A. CLASSIFICATION OF SUBJECT MATTER |
| --- |
| Int. Cl. A61B5/1455(2006.01)i, G01N33/49(2006.01)n |

According to International Patent Classification (IPC) or to both national classification and IPC

| B. FIELDS SEARCHED |
| --- |
| Minimum documentation searched (classification system followed by classification symbols) |
| Int. Cl. A61B5/1455, G01N33/49 |

Documentation searched other than minimum documentation to the extent that such documents are included in the fields searched

```
Published examined utility model applications of Japan      1922-1996
Published unexamined utility model applications of Japan    1971-2018
Registered utility model specifications of Japan            1996-2018
Published registered utility model applications of Japan    1994-2018
```

Electronic data base consulted during the international search (name of data base and, where practicable, search terms used)

| C. DOCUMENTS CONSIDERED TO BE RELEVANT |
| --- |

| Category* | Citation of document, with indication, where appropriate, of the relevant passages | Relevant to claim No. |
| --- | --- | --- |
| Y | JP 2004-248716 A (CITIZEN WATCH CO., LTD.) 09 September 2004, entire text, fig. 1 (Family: none) | 1-12 |
| Y | ZHERNOVAYA O.S. et al., Monitoring of blood proteins glycation by refractive index and spectral measurements, Laser Physics Letters, 28 February 2008, Volume 5, Number 6, pp. 460-464 | 1-12 |

☐ Further documents are listed in the continuation of Box C.    ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| | |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005253478 A
- JP 2004248716 A
- EP 0631137 A
- JP H07088105 A
- WO 03071939 A
- JP 2004081284 A
- JP 3875798 B
- JP H11155840 A
- JP 2008546430 A

**Non-patent literature cited in the description**

- *Laser Physics Letters,* 2008, vol. 5, 460-464